# EUROPEAN PATENT SPECIFICATION

(11) **EP 2 155 273 B1**
(45) Date of publication and mention of the grant of the patent: **16.01.2013**
(21) Application number: 08770609.9
(22) Date of filing: 10.06.2008
(51) Int. Cl.: A61L 15/46, A61L 15/58, A61L 15/60

(54) **ODOR-CONTROL ARTICLE**
GERUCHSREGELUNGSARTIKEL
ARTICLE PERMETTANT DE RÉGULER LES ODEURS

(30) Priority: 11.06.2007 US 943137 P
(43) Date of publication of application: 24.02.2010
(73) Proprietor: Avery Dennison Corporation, Pasadena, California 91103 (US)
(72) Inventor: VAN BAVEL, Davy, B-2350 Vosselaar (BE); WARD, Colleen, B-2350 Vosselaar (BE); WIBAUX, Anne Marie Paule, B-2018 Antwerpen (BE)
(74) Representative: Ilgart, Jean-Christophe
(86) International application number: PCT/US2008/066443
(87) International publication number: WO 2008/154546

(56) References cited:
- WO-A-00/16752
- US-A1- 2006 041 987
- US-A1- 2006 251 609

## Description

### FIELD OF THE INVENTION

The present invention generally relates to a disposable, self-adherent odor-control article, and more particularly, to an odor-absorbent patch that adheres directly to the skin of the user.

### BACKGROUND

Perspiring is a natural bodily function that primarily is a means of maintaining the body's temperature. Perspiration consists mainly of water and small amounts of salt, urea, sugar, and ammonia. There are two types of glands in the underarm, the apocrine and eccrine. The eccrine glands are by far the most numerous sweat glands and are responsible for producing most of the sweat in the underarms, as well as in the entire body. The apocrine sweat glands present in the underarm produce sweat that also contains proteins and fatty acids. Sweat itself is not generally malodorous. It is the bacteria that break down the organic compounds in the sweat that causes malodors.

Deodorants are often applied to the skin to cover up underarm malodors, and antiperspirants are used to slow down the production of underarm sweat. Deodorants and antiperspirants can be applied to the skin in the form of aerosols, roll-ons, solid sticks, and gels. While the use of these products can mask odors and/or reduce the production of sweat, they are not entirely effective in eliminating malodors, particularly when there is profuse sweating due to physical exertion, heat, stress, anxiety, or hyperhydrosis. Also, deodorants and/or antiperspirants do not address the problem of staining of clothing, and can in fact add to the discoloration or damage caused to the user's garment.

In order to protect garments from the effects of perspiration, absorbent shields have been applied to the underarm area of clothing. Generally such shields contain an absorbent cloth-like outer layer and an inner adhesive layer that is detachably secured to the article of clothing at the underarm seam. While these shields may protect the garment from discoloration and staining, they often are bulky and may become dislodged. In addition, because the shield is remote from the source of the perspiration, odor causing bacteria has access to the organic compounds in the perspiration.

Underarm shields that are applied directly to the user's skin have been developed. For example, U.S. Patent Application Publication Number 2006/0041987 to Villain discloses a disposable patch constructed of a first body-contacting layer made of a porous, moisture-permeable material with an adhesive strip around the periphery, a second inner layer made from a high moisture-absorbing material with an impregnated scent, a third layer made from a moisture-impermeable material. U.S. Patent Number 3,885,247 to Kost discloses a self-adhering disposable protector that includes a moisture-proof backing, and a moisture-absorbing pad secured to one side of the backing for absorbing perspiration. The pad includes an absorbent filler material and a hydrophobic inner shield that is disposed on, and covers, one side of the filler material opposite from the backing for contact with the wearer's skin. The backing includes outwardly projecting tabs that include an adhesive material for holding the inner shield to the wearer's underarm. The inner shield includes an adhesive material along its periphery for attaching the inner shield to the wearer's body.

US 2006/0251609 discloses a patch for use in conjunction with a skin surface which comprises
- a translucent backsheet which is intended to be applied to the skin, which has therefore an adherent surface and which may be breathable; and
- a cover sheet which may be more breathable than the backsheet; and which may comprise a scent pad that could absorb the moisture, in which case the scent pad is interposed between the backsheet and the cover sheet.

While such shields may be absorbent, the shields do not prevent bacteria from interacting with the perspiration. Furthermore, these shields are adhered to the user's skin with a limited area of adhesive, generally around just the periphery of the absorbent pad, and therefore may not be securely adhered to the skin, particularly if the user is profusely perspiring.

### SUMMARY

The patch of the present invention includes a thin laminate that can be securely adhered directly to the skin of the user. The direct contact between the adhesive layer and the user's skin reduces the potential interaction between bacteria and the organic components of the perspiration.

An exemplary embodiment is a patch that is adapted to receive perspiration and control malodors associated therewith. The patch includes a skin-adherent adhesive layer having a pressure sensitive adhesive and at least one odor-control agent, a flexible backing layer and a fluid absorbent pad that overlies at least a portion of the skin-adherent adhesive layer.

In other, more detailed features of the invention, the pressure sensitive adhesive is a rubber-based adhesive, an acrylic adhesive, a silicone adhesive, or a polyurethane adhesive. Also, the pressure sensitive adhesive can include a composition that is a styrene-isoprene-styrene polymer, a styrene-olefin-styrene polymer, polyisobutylene, a styrene-butadiene-styrene polymer, polyisoprene, polybutadiene, a natural rubber, a silicone rubber, an acrylonitrile rubber, a nitrile rubber, a polyurethane rubber, a polyisobutylene rubber, a butyl rubber, a halobutyl rubber, a butadiene-acrylonitrile rubber, polychloroprene, a styrene-butadiene rubber, or a combination thereof.

The adhesive layer of the patch contains at least one fluid-absorbing agent. The fluid-absorbing agent absorbs the perspiration, thus reducing, or eliminating, the damage done to the user's garment and the appearance of wetness on the exterior of the garment.

In other, more detailed features of the invention, the fluid-absorbing agent is a hydrocolloid, carboxy methyl cellulose, or polyacrylate superabsorbent. Also, the odor-control agent can be absorbent. In addition, the flexible backing layer can include a polymeric film. Furthermore, the polymeric film can include polyurethane.

In other, more detailed features of the invention, the flexible backing layer is made of a non-woven material. Also, the odor-control agent can include a composition that is activated carbon, clay, zeolite, silica, cyclodextrin, or a combination thereof. In addition, the flexible backing layer can have a thickness that is less than about 500 micrometers, or less than about 100 micrometers. Furthermore, the skin-adherent adhesive layer can have a thickness that is from about 20 micrometers to about 1 millimeter.

In other, more detailed features of the invention, the patch also includes another skin-adherent pressure sensitive adhesive layer between the skin-adherent adhesive layer and the flexible backing layer. The skin-adherent adhesive layer is characterized as having a first adhesive strength. The another skin-adherent adhesive layer is characterized as having a second adhesive strength. The second adhesive strength is greater than the first adhesive strength.

### BRIEF DESCRIPTION OF THE DRAWINGS

These and other features, aspects, and advantages of the present invention will become better understood with reference to the following description, appended claims, and accompanying drawings, where:
FIG. 1 is a perspective view showing a typical application of the underarm patch as applied to the skin of the user according to a comparative embodiment.
FIG. 2 is a sectional view of one comparative embodiment of the underarm patch.
FIG. 3 is a sectional view of another comparative embodiment of the underarm patch where the patch includes a removable liner.
FIG. 4 is a top plan view of an alternative comparative embodiment of the underarm patch having a first odor-control adhesive layer and a second pressure adhesive layer.
FIG. 5 is a sectional view of the embodiment of the underarm patch of FIG. 4.
FIG. 6 is a top plan view of an embodiment of the underarm patch having a fluid-absorbing layer, a first odor-control adhesive layer and a second pressure sensitive adhesive layer.
FIG. 7 is a sectional view of the embodiment of the underarm patch of FIG. 6.

Unless otherwise indicated, the illustrations in the above figures are not necessarily drawn to scale.

### DETAILED DESCRIPTION

The disposable, flexible underarm patch of the present invention includes a thin perspiration barrier that controls malodors associated with perspiration. Because the patch is securely adhered directly to the underarm skin of the user, there is little opportunity for airborne or surface bacteria to access the organic components of the perspiration and cause malodors.

According to the invention, an underarm patch is adapted to receive perspiration and control malodors associated therewith including the following: a skin-adherent adhesive layer including a pressure sensitive adhesive and at least one odor-control agent, and a flexible backing layer.

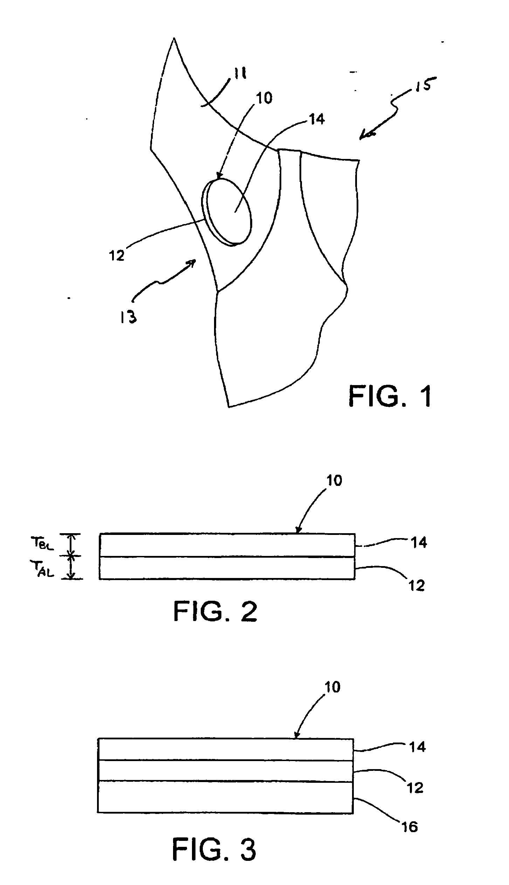

Referring now to the drawings in detail, and initially to FIG. 1, an underarm patch 10 is illustrated as adhered to the skin 11 of the user 15. The patch is worn such that the patch's adhesive layer 12 is adhered to the skin in the underarm area 13 with the flexible backing layer 14 facing outward.

FIG. 2 illustrates an embodiment of the underarm patch 10 that includes the adhesive layer 12 adhered directly to the flexible backing layer 14. A release liner 16 can optionally be removably adhered to the adhesive layer to protect the adhesive layer prior to use, as illustrated in FIG. 3.

The adhesive layer 12 of the invention can include any suitable pressure sensitive adhesive matrix known in the art that can be applied directly to skin 11. The permanently tacky pressure sensitive adhesive component must be tacky at room temperature as well as at the skin temperature of users. Also, the adhesive must be dermatologically acceptable, which means that after continuous contact with skin there is little adhesive residue upon removal and there is no significant reaction with the skin during the adhesion period.

The adhesive strength of the adhesive layer 12 must be sufficient to adhere to the skin 11 of the user 15 for the time determined by the use of the odor-absorbing article 10. Other ingredients for example, tackifiers, plasticizers, and polymer stabilizers can be added to the adhesive to modify tack and optimize adhesion properties and to protect polymers from degradation during processing.

The adhesive matrix can be based on, for example, acrylic, rubber, polyurethane, silicone, or polyvinyl ether-based adhesives. Examples of polymeric rubber-based adhesives include one or more styrene-isoprene-styrene polymers, styrene-olefin-styrene polymers including styrene-ethylene/propylene-styrene polymers, polyisobutylene, styrene-butadiene-styrene polymers, polyisoprene, polybutadiene, natural rubber, silicone rubber, acrylonitrile rubber, nitrile rubber, polyurethane rubber, polyisobutylene rubber, butyl rubber, halobutyl rubber including bromobutyl rubber, butadiene-acrylonitrile rubber, polychloroprene and styrene-butadiene rubber. Blends or mixtures of elastomers can be employed.

Conventional additives, for example, tackifiers, softeners, plasticizers, and antioxidants can be present to modify, adjust, and stabilize the adhesive and other properties of the matrix. Tackifiers are generally hydrocarbon resins, wood resins, rosins, rosin derivatives, and the like. It is contemplated that any tackifier known by those skilled in the art to be compatible with the adhesive matrix can be used.

The amount of adhesive matrix with respect to the total composition will generally be from 35 wt% to about 99 wt%, or more. Hydrophilic polymers, for example, as used in hydrogels, also can form the basis of the pressure sensitive adhesive matrix.

The adhesive matrix includes at least one fluid-absorbing agent. An example of a fluid-absorbing agent is polyacrylate superabsorbent. The fluid-absorbing agent can include hydrocolloid particles dispersed, or dissolved, within the adhesive matrix. Suitable hydrocolloids for use in the adhesives in conjunction with the odor-absorbing agent are naturally occurring hydrocolloids, e.g., pectins, guar gum, karaya gum, locust bean gum, carageenan, tragacanth gum, alginates, xanthan gum, modified naturally derived substances, e.g., sodium carboxymethyl cellulose, synthetic materials, e.g., polvinylalcohol, polyoxyalkylene polyols, polyvinyl pyrollidone, and animal derived materials, e.g., gelatine.

Ionic hydrocolloids, for example, hyaluronic acid, chitosan salts or DEAE Dextran also can be employed. The hydrocolloids can be water absorbable or water swellable, and combinations of one type, or of various types, can be used in any ratio. A hydrocolloid can be used in an amount from 0 wt% to 60 wt%, or more.

The adhesive layer 12 includes at least one odor-control agent. Odors can be classified as acidic, basic, or neutral. Acidic odor-controlling agents have a pH greater than 7, and typically include sodium carbonates, sodium bicarbonates, sodium phosphates, particularly zinc and copper sulphates. Basic odor-controlling agents have a pH of less than 7, and include compounds, for example, carboxylic acids, e.g., citric acid, laric acid, boric acid, adipic acid, and maleic acid. Neutral odor-controlling agents have a pH or approximately 7. Examples of these compounds include activated carbons, days, zeolites, silicas, absorbent gelling materials, and starches. Cyclodextrin is a particularly useful odor-control agent.

The term cyclodextrin, as used herein, includes any of the known cyclodextrins. Cyclodextrin materials are cyclic oligosaccharides containing a minimum of six D-(+)- glucopyranose units attached by α- (1 > 4) glucosidic bonds.

Three cyclodextrins called α, β, and γ are naturally occurring and have, respectively, six, seven, and eight glucose units. Cyclodextrin's are known that contain up to twelve glucose units. Cyclodextrin materials can also be manufactured from starch by enzymatic degradation. In addition, many synthetic modifications of the natural material are known, for example methyl-β-cyclodextrin and hydroxypropyl-β-cyclodextrin. The conformations of the cyclic structures of these molecules are such that the molecules are arranged in rigid conical molecular shapes that have hollow interiors of very well defined sizes. These internal cavities are hydrophobic in nature because the interior of the toroidal shape is predominantly made up of hydrogen atoms. The interior shapes of the cyclodextrins are able to form inclusion complexes, sometimes referred to as "host-guest" complexes, or clathrate compounds, with organic molecules that fit, completely or partially, into the cavities defined by the toroidal shapes. For example, odiferous molecules can fit into the cavities. This includes both perfumes and malodorous compounds. Cyclodextrins therefore, and especially mixtures of cyclodextrins with cavities of different sizes, can be used to control odors. With respect to odor control, there is scope for two different approaches within the present invention.

First, uncomplexed or free cyclodextrins, dispersed within the adhesive matrix, can be used to absorb malodours. Second, perfumes can be precomplexed with cyclodextrins, and then, formulated in the final adhesive. The perfume is then released in situ and will mask the undesirable odor. (Once a cyclodextrin molecule has released its precomplexed perfume molecule, it is then available to complex a malodorous molecule). Complexed cyclodextrins also can be included in the adhesive. The complexation of odorous molecules by cyclodextrin and the release of precomplexed perfume molecules from cyclodextrin are facilitated by the presence of water. It will be understood that the water necessary to facilitate such a release of perfume and complexing of malodour by the cyclodextrin, is present in the perspiration, and will be absorbed by the adhesive.

The choice of cyclodextrin employed in a given formulation will be decided on the basis of the properties desired in the finished product, and the specific role that the cyclodextrin is fulfilling. Unmodified β-cyclodextrin is not very water soluble and is generally not preferred if high absorbency is needed (α-cyclodextrins, cyclodextrins, and certain modified β-cyclodextrins are more water absorbent).

Mixtures of cyclodextrins are often preferred, because these will absorb a wider range of malodorous molecules than will a single cyclodextrin. The cyclodextrin to be used for a specific complex will of course be determined by the size and shape of the active molecule to be complexed.

In addition to the odor-control agent, the adhesive layer 12 can contain additional ingredients, for example, antimicrobial agents, antiperspirants, fragrances, and skin protectants. Examples of antimicrobial agents include silver, copper, and bacterial and/or fungicidal compounds Antiperspirant compounds include astringent metallic salts, especially the inorganic and organic salts of aluminum, zirconium and zinc, as well as mixtures thereof. Aluminum-containing and/or zirconium-containing materials or salts include aluminum halides, aluminum chlorohydrate, aluminum hydroxyhalides, zirconyl oxyhalides, zirconyl hydroxyhalides, and mixtures thereof. Also included are aluminum-zirconium tetrachlorohydrex and aluminum-zirconium trichlorohydrex.

The adhesive layer 12 can be coated directly onto the backing layer 14, or can be transferred to the backing layer from a transfer tape or film. In one embodiment, the adhesive layer is coated onto a release liner 16 and then adhered to the backing layer. Any suitable release liner know to those skilled in the art can be used to protect the adhesive layer prior to use of the underarm patch 10. The thickness "T_{AL}" of the adhesive layer is generally less than about 1 millimeter. In one embodiment, the thickness of the adhesive layer "T_{AL}" is about 20 micrometers to about 1 millimeter. In another embodiment, the thickness of the adhesive layer "T_{AL}" is about 50 micrometers to about 75 micrometers. In one embodiment, the adhesive layer is a continuous layer on the backing layer. The adhesive can be applied to a major central portion of the backing film, such that the backing film has the same general shape as the adhesive layer but is slightly larger, leaving an adhesive free area around at least a portion of the periphery of the backing layer.

The material of the flexible backing layer 14 material can be any suitable polymeric film, plastic foam (including open celled foam), a woven fabric, a knitted fabric, or a non-woven fabric. The fabrics can be made from natural or synthetic materials. As used herein, the term "flexible" refers to materials that are compliant, and that will readily conform to the general shape and contours of the human body. The backing layer preferably possesses at least some breathability.

In one embodiment, a porous backing layer 14 is employed. For example, in one embodiment, the backing layer is an apertured polymeric film, for example, polyolefin film, a non-woven, cloth fabric, or the like.

Examples of materials suitable for use as a backing layer 14 or strip include polyolefins, e.g., polyethylene, polypropylene, ethylene propylene copolymers, and ethylene butylene copolymers, polyurethanes, polyurethane foams, polystyrenes, plasticized polyvinylchlorides, polyesters, polyamides, and cotton. The plastic film can be in the form of a sheet or foam strip. Specific examples include a polyurethane film having a thickness of about 25 microns. Another useful backing layer is an 83.8 micron apertured polyolefin film.

The backing layer 14 is preferably flexible yet resistant to tearing. In one embodiment, the thickness of the backing layer "T_{BL}" of the adhesive article 10 of the present invention is less than 500 micrometers, or less than 300 micrometers, or less than 100 micrometers. In one embodiment the thickness of the backing layer "T_{BL}" is from about 15 microns to about 500 microns. In another embodiment, the thickness of the backing layer "T_{BL}" is from about 20 microns to about 300 microns. In another embodiment, the thickness of the backing layer "T_{BL}" is from about 25 microns mil to about 100 microns.

The backing layer 14 can be opaque or translucent. In one embodiment, the backing layer has a skin color. The backing layer can be solid or porous, permeable or perforated, as adapted for the requirements of the product application, as well as being a function of the composition and form of the backing material. In one embodiment, the adhesive article 10 is pigmented.

In one embodiment, the backing layer 14 is substantially impervious to liquid, especially perspiration. In yet another embodiment, the backing layer is substantially impervious to bacteria. In another embodiment, the backing layer is capable of absorbing liquid, especially perspiration. In another embodiment, perforations or small apertures in the backing layer facilitate high breathability.

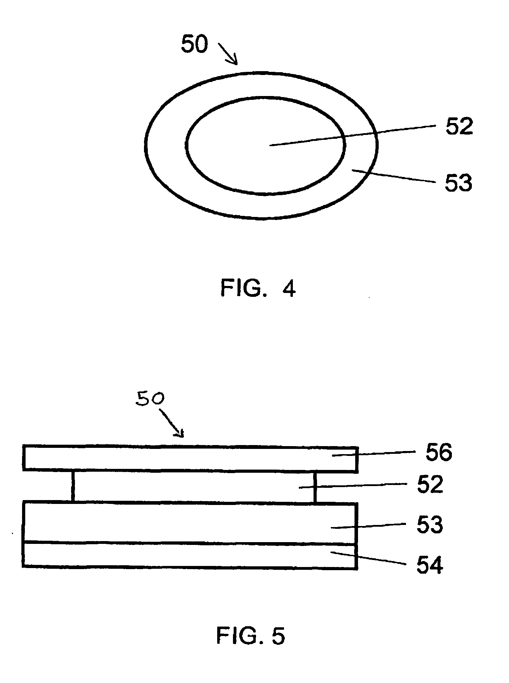

Referring to FIGS. 4 and 5, the underarm patch 50 having an odor-control adhesive layer 52 overlying a pressure sensitive adhesive layer 53, which is adhered to backing layer 54 is illustrated. The adhesive layer extends beyond the outer periphery of the odor-control adhesive layer. The adhesive layer includes a pressure sensitive adhesive having a greater adhesive strength than that of the odor-control adhesive layer. An optional release liner 56 can be adhered to the odor-control adhesive layer and the region of adhesive layer that extends beyond adhesive layer in order to protect the adhesive layers prior to use.

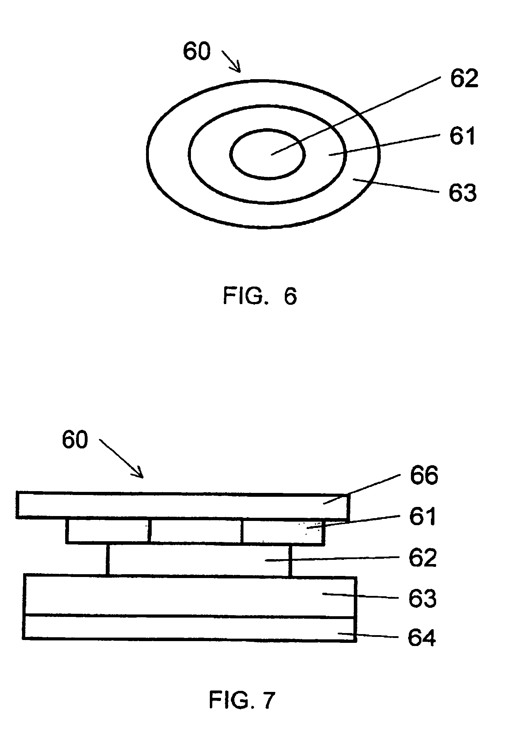

Referring to FIGS. 6 and 7, an embodiment of the under arm patch 60 having an odor-control adhesive layer 62 overlying a pressure sensitive adhesive layer 63, which is adhered to backing layer 64 is illustrated. Overlying a portion of odor-control adhesive layer is a fluid-absorbent pad 61. The fluid-absorbent pad 61 can include any absorbent material known in the art for absorbing bodily fluids. These materials include nonwovens, textiles, water swellable polymers, etc. The absorbent pad provides additional comfort and absorption to the underarm patch. The absorbent pad coverts a portion of the odor-control adhesive layer, leaving another portion exposed so that the odor-control adhesive directly contacts the underarm skin 11. The adhesive layer extends beyond the outer periphery of odor-control adhesive layer As described above, adhesive layer is a pressure sensitive adhesive layer having a greater adhesive strength than that of odor-control adhesive layer. An optional release liner 66 can be adhered to the exposed portions of the adhesive layers to protect the adhesive layers prior to use.

### Example (comparative)

An underarm patch 10 is constructed by applying a 50 micron to 1 millimeter thick layer 12 of an adhesive composition of styrene-isoprene-styrene and polyisobutylene polymers with carboxy methyl cellulose dispersed therein onto a 25 micron thick polyurethane backing 14. A release liner 16 is then applied to the adhesive layer to protect the adhesive layer prior to use.

## Claims

1. A patch that is adapted to receive perspiration and control malodors associated therewith, the patch comprising:
a skin-adherent adhesive layer including a pressure sensitive adhesive and at least one odor-control agent;
a flexible backing layer; and
a fluid-absorbent pad that overlies a portion of the skin adherent adhesive layer.

2. The patch according to claim 1, wherein the pressure sensitive adhesive is selected from the group consisting of a rubber-based adhesive, an acrylic adhesive, a silicone adhesive, and a polyurethane adhesive.

3. The patch according to claim 2, wherein the pressure sensitive adhesive includes a composition selected from the group consisting of a styrene-isoprene-styrene polymer, a styrene-olefin-styrene polymer, polyisobutylene, a styrene-butadiene-styrene polymer, polyisoprene, polybutadiene, a natural rubber, a silicone rubber, an acrylonitrile rubber, a nitrile rubber, a polyurethane rubber, a polyisobutylene rubber, a butyl rubber, a halobutyl rubber, a butadiene-acrylonitrile rubber, polychloroprene, a styrene-butadiene rubber, and a combination thereof.

4. The patch according to claim 1, further comprising at least one fluid-absorbing agent.

5. The patch according to claim 4, wherein the fluid-absorbing agent is selected from the group consisting of a hydrocolloid, carboxy methyl cellulose, and polyacrylate superabsorbent.

6. The patch according to claim 1, wherein the odor-control agent is absorbent.

7. The patch according to claim 1, wherein the flexible backing layer includes a polymeric film.

8. The patch according to claim 7, wherein the polymeric film includes polyurethane.

9. The patch according to claim 1, wherein the flexible backing layer is made of a non-woven material.

10. The patch according to claim 1, wherein the odor-control agent includes a composition selected from the group consisting of activated carbon, clay, zeolite, silica, cyclodextrin, and a combination thereof.

11. The patch according to claim 1, wherein the flexible backing layer has a thickness that is less than 500 micrometers.

12. The patch according to claim 1, wherein the flexible backing layer has a thickness that is less than 100 micrometers.

13. The patch according to claim 1 wherein the skin-adherent adhesive layer has a thickness that is from 20 micrometers to 1 millimeter.

14. The patch according to claim 1, further comprising another skin-adherent pressure sensitive adhesive layer between the skin-adherent adhesive layer and the flexible backing layer, wherein:
the skin-adherent adhesive layer is characterized as having a first adhesive strength;
the another skin-adherent adhesive layer is characterized as having a second adhesive strength; and
the second adhesive strength is greater than the first adhesive strength.

## Patentansprüche

1. Pflaster, das ausgebildet ist, um Schweiß aufzunehmen und diesem zugehörige schlechte Gerüche zu regulieren, wobei das Pflaster aufweist:
eine an Haut haftende Klebstoffschicht, die einen haftklebenden Klebstoff und mindestens ein Geruchsreguliermittel beinhaltet;
eine flexible Rückenschicht; und
ein fluidabsorbierendes Polster, das einen Abschnitt der an Haut haftenden Klebstoffschicht überlagert.

2. Pflaster nach Anspruch 1, wobei der haftklebende Klebstoff aus der Gruppe gewählt ist, die aus einem gummibasierten Klebstoff, einem Acrylklebstoff, einem Silikonklebstoff und einem Polyurethanklebstoff besteht.

3. Pflaster nach Anspruch 2, wobei der haftklebende Klebstoff eine Zusammensetzung beinhaltet, die aus der Gruppe gewählt ist, welche aus einem StyroI-Isopren-Styrolpolymer, einem Styrol-Olefin-Styrolpolymer, Polyisobutylen, einem Styrol-Butadien-Stryrolpolymer, Polyisopren, Polybutadien, einem Naturkautschuk, einem Silikonkautschuk, einem Acrylonitril-Kautschuk, einem Nitril-Kautschuk, einem Polyurethan-Kautschuk, einem Polyisobutylen-Kautschuk, einem Butyl-Kautschuk, einem Halobutyl-Kautschuk, einem Butadien-Acrylonitril-Kautschuk, Polychloropren, einem Styrol-Butadien-Kautschuk, und einer Kombination von diesen besteht.

4. Pflaster nach Anspruch 1, das weiter mindestens ein fluidabsorbierendes Mittel beinhaltet.

5. Pflaster nach Anspruch 4, wobei das fluidabsorbierende Mittel aus der Gruppe gewählt ist, welche aus einem Hydrokolloid, Carboxymethylcellulose und einem Polyacrylat-Superabsorber besteht.

6. Pflaster nach Anspruch 1, wobei das geruchsregulierende Mittel absorbierend ist.

7. Pflaster nach Anspruch 1, wobei die flexible Rückenschicht einen Polymerfilm beinhaltet.

8. Pflaster nach Anspruch 7, wobei der Polymerfilm Polyurethan beinhaltet.

9. Pflaster nach Anspruch 1, wobei die flexible Rückenschicht aus einem Vliesmaterial besteht.

10. Pflaster nach Anspruch 1, wobei das geruchsregulierende Mittel eine Zusammensetzung beinhaltet, die aus der Gruppe gewählt ist, welche aus Aktivkohle, Ton, Zeolith, Silica, Cyclodextrin, und einer Kombination von diesen besteht.

11. Pflaster nach Anspruch 1, wobei die flexible Rückenschicht eine Dicke hat, die weniger als 500 Mikrometer beträgt.

12. Pflaster nach Anspruch 1, wobei die flexible Rückenschicht eine Dicke hat, die weniger als 100 Mikrometer beträgt.

13. Pflaster nach Anspruch 1, wobei die an Haut haftende Klebstoffschicht eine Dicke von 20 Mikrometer bis 1 Millimeter hat.

14. Pflaster nach Anspruch 1, das weiter eine weitere an Haut haftende haftklebende Klebstoffschicht zwischen der an Haut haftenden Klebstoffschicht und der flexiblen Rückenschicht aufweist, wobei:
die an Haut haftende Klehstoffschicht durch eine erste Klebefestigkeit gekennzeichnet ist;
die weitere an Haut haftende Klebstoffschicht durch eine zweite Klebefestigkeit gekennzeichnet ist; und
die zweite Klebefestigkeit größer als die erste Klebefestigkeit ist.

## Revendications

1. Timbre qui est adapté pour recevoir la transpiration et réguler les mauvaises odeurs qui y sont associées, le timbre comprenant :
une couche adhésive adhérant à la peau incluant un adhésif sensible à la pression et au moins un agent de régulation des odeurs ;
une couche de support flexible ; et
un tampon absorbant les fluides qui recouvre une portion de la couche adhésive adhérant à la peau.

2. Timbre selon la revendication 1, dans lequel l'adhésif sensible à la pression est choisi dans le groupe constitué par un adhésif à base de caoutchouc, un adhésif acrylique, un adhésif silicone et un adhésif polyuréthanne.

3. Timbre selon la revendication 2, dans lequel l'adhésif sensible à la pression inclut une composition choisie dans le groupe constitué par un polymère styrène-isoprène-styrène, un polymère styrène-oléfine-styrène, un polyisobutylène, un polymère styrène-butadiène-styrène, un polyisoprène, un polybutadiène, un caoutchouc naturel, un caoutchouc silicone, un caoutchouc acrylonitrile, un caoutchouc nitrile, un caoutchouc polyuréthanne, un caoutchouc polyisobutylène, un caoutchouc butyle, un caoutchouc halogénobutyle, un caoutchouc butadiène-acrylonitrile, un polychloroprène, un caoutchouc styrène-butadiène, et une combinaison de ceux-ci.

4. Timbre selon la revendication 1, comprenant en outre au moins un agent absorbant les fluides.

5. Timbre selon la revendication 4, dans lequel l'agent absorbant les fluides est choisi dans le groupe constitué par un hydrocolloïde, une carboxyméthylcellulose et un superabsorbant en polyacrylate.

6. Timbre selon la revendication 1, dans lequel l'agent de régulation des odeurs est absorbant.

7. Timbre selon la revendication 1, dans lequel la couche de support flexible inclut un film polymère.

8. Timbre selon la revendication 7, dans lequel le film polymère inclut du polyuréthanne.

9. Timbre selon la revendication 1, dans lequel la couche de support flexible est constituée d'un matériau non tissé.

10. Timbre selon la revendication 1, dans lequel l'agent de régulation des odeurs inclut une composition choisie dans le groupe constitué par le charbon actif, l'argile, la zéolite, la silice, la cyclodextrine, et une combinaison de ceux-ci.

11. Timbre selon la revendication 1, dans lequel la couche de support flexible a une épaisseur qui est inférieure à 500 micromètres.

12. Timbre selon la revendication 1, dans lequel la couche de support flexible a une épaisseur qui est inférieure à 100 micromètres.

13. Timbre selon la revendication 1, dans lequel la couche adhésive adhérant à la peau a une épaisseur qui est de 20 micromètres à 1 millimètre.

14. Timbre selon la revendication 1, comprenant en outre une autre couche adhésive sensible à la pression adhérant à la peau entre la couche adhésive adhérant à la peau et la couche de support flexible, dans lequel :
la couche adhésive adhérant à la peau est **caractérisée en ce qu'**elle a une première force d'adhésion ;
faute couche adhésive adhérant à la peau est **caractérisée en ce qu'**elle a une seconde force d'adhésion ; et
la seconde force d'adhésion est supérieure à la première force d'adhésion.
